(19) Europäisches Patentamt
European Patent Office
Office européen des brevets

(11) EP 4 037 640 B1

(12) EUROPEAN PATENT SPECIFICATION

(45) Date of publication and mention of the grant of the patent:
**15.03.2023 Bulletin 2023/11**

(21) Application number: **20753384.5**

(22) Date of filing: **13.08.2020**

(51) International Patent Classification (IPC):
***A61K 8/24*** (2006.01) ***A61K 8/90*** (2006.01)
***A61Q 11/00*** (2006.01) ***A61K 8/04*** (2006.01)

(52) Cooperative Patent Classification (CPC):
**A61K 8/90; A61K 8/046; A61K 8/24; A61Q 11/00**

(86) International application number:
**PCT/EP2020/072821**

(87) International publication number:
**WO 2021/063581 (08.04.2021 Gazette 2021/14)**

# (54) AN ORAL CARE SPRAY COMPOSITION COMPRISING HEXAMETAPHOSPHATE

MUNDPFLEGESPRAYZUSAMMENSETZUNG ENTHALTEND HEXAMETAPHOSPHAT

COMPOSITION DE SPRAY DE SOIN ORAL COMPRENANT DE L'HEXAMETAPHOSPHATE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.10.2019 EP 19201369**

(43) Date of publication of application:
**10.08.2022 Bulletin 2022/32**

(73) Proprietors:
- **Unilever Global IP Limited**
  **Wirral, Merseyside CH62 4ZD (GB)**
  Designated Contracting States:
  **CY DE GB IE IT MT RS TR**
- **Unilever IP Holdings B.V.**
  **3013 AL Rotterdam (NL)**
  Designated Contracting States:
  **AL AT BE BG CH CZ DK EE ES FI FR GR HR HU IS LI LT LU LV MC MK NL NO PL PT RO SE SI SK SM**

(72) Inventors:
- **ALLAHBASH, Shahin**
  **Whitefield, Bangalore 560 066 (IN)**
- **BARNE, Sameer, Keshav**
  **Whitefield, Bangalore 560 066 (IN)**
- **DABHOLKAR, Nandini, Sachin**
  **Mumbai 400099 Andheri (IN)**
- **JOINER, Andrew**
  **Wirral Merseyside CH63 3JW (GB)**
- **JOSHI, Aditi, Balkrishna**
  **Mumbai 400099 Andheri (IN)**
- **PHILPOTTS, Carole, Jane**
  **Wirral Merseyside CH63 3JW (GB)**
- **SRIDHAR, Lakshmy**
  **Mumbai 400099 Andheri (IN)**
- **VAIDYA, Ashish, Anant**
  **Whitefield, Bangalore 560 066 (IN)**

(74) Representative: **Tansley, Sally Elizabeth**
**Unilever Patent Group**
**Bronland 14**
**6708 WH Wageningen (NL)**

(56) References cited:
**US-A- 4 370 314** **US-A- 5 057 309**
**US-A1- 2013 344 009**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 037 640 B1

## Description

### Field of the Invention

The present invention relates to an oral care composition for minimising staining of teeth.

### Background of the Invention

Long-lasting whitening of teeth is of considerable interest to consumers. Foods and drinks such as tea, coffee and wine may form dental stains by directly depositing chromogens on the tooth surface. Attraction of materials to the tooth surface plays a critical role in the deposition of extrinsic dental stain. The chromogens in these beverages that are responsible for causing dental stain are known as tannins and are composed of polyphenols such as catechins. These materials generate colour due to the presence of conjugated double bonds.

Traditional tooth whitening methods involve either peroxide bleaching from kit formats or abrasive stain removal from toothpaste formats. These particles are essentially insoluble particles that remove extrinsic stain from the surface of the tooth via abrasion when applied with a brush. The present invention relates to a novel oral care composition that delivers effective levels of stain protection and prolongs the effects of tooth whitening by preventing staining of teeth. The invention discloses a stain repellent coating having a synergistic blend of a hydrophilic-hydrophobic block co-polymer and $Ca^{2+}$ chelating agent (a phosphate salt) at a defined ratio. This helps to provide a protective stain coating on the tooth surface during the consumption of food/drinks post whitening treatments.

The block copolymers used in the present invention are what are generally known as Pluronics. These polymers, in general, have been known to be used in oral care. The phosphate salt used in the present invention is hexametaphosphate which has also been used in oral care.

US2014377194A (P&G) discloses an oral care composition containing (a) a zinc citrate; and (b) a surface-active organophosphate compound and it also relates to a method of preventing a stain from depositing on a tooth surface or other oral surfaces.

US2003124065A (P&G) discloses an oral care composition and methods for overall cleaning, whitening and preventing, reducing or removing surface deposited stains on natural teeth and dental prosthesis, the compositions comprising in an orally acceptable carrier at least 0.1 % by weight of a water-soluble or water-dispersible copolymer prepared by copolymerizing one or a mixture of vinyl pyrrolidone (VP) monomers with one or a mixture of C1-C19 alkyl carboxylic acid (AC) C2-C12 alkenyl ester monomers; preferably, these compositions further comprise one or a mixture of other oral care agents selected from a water soluble alkali metal or ammonium tripolyphosphate in an amount at least about 0.5% by weight of the composition, an abrasive, preferably a precipitated silica abrasive, in an amount at least about 6% by weight of the composition and a bleaching agent in an amount at least about 0.1% by weight of the composition. US5057309B discloses an oral spray comprising Pluronic.

It is thus an object of the present invention to provide for an oral care composition that minimises staining of teeth.

### Description of the Invention

According to the first aspect of the present invention there is provided an oral care composition comprising:

(a) 0.1 to 2 wt% of the total composition of polymer having the structure

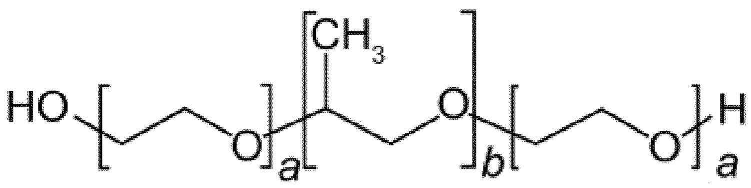

in which a is independently selected from 2 to 130 and b is 15 to 67 and the molecular weight of the polymer is from 1700 to 15000 Da.

(b) hexametaphosphate; and

(c) an orally acceptable base;

in which the weight ratio of the polymer to hexametaphosphate is from 1:5 to 5:1,

According to another aspect of the present invention there is provided a method of minimising or preventing the staining of teeth comprising the steps of applying the composition of the invention described above on to a tooth.

A further aspect of the invention relates to cosmetic use of a composition according to any preceding claim to mitigate the staining of teeth.

## Detailed Description of the Invention

Except in the examples, or where otherwise explicitly indicated, all numbers in this description indicating amounts of material or conditions of reaction, physical properties of materials and/or use may optionally be understood as modified by the word "about".

All amounts are by weight of the final composition, unless otherwise specified.

It should be noted that in specifying any ranges of values, any particular upper value can be associated with any particular lower value.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

The present invention relates to an oral care composition comprising a polymer having the structure of compound of formula 1.

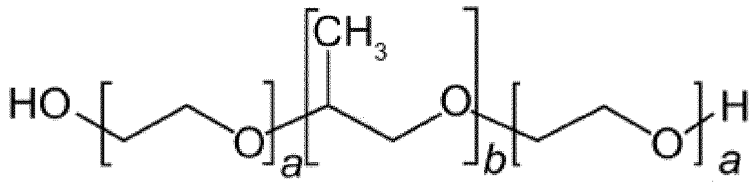

Compound of formula 1.

In the above compound, the molecular weight ratio of ethoxylate groups to the propoxylate groups is in the range of 0.1 to 3.0, preferably in the range of 0.1 to 2.5. The molecular weight of the polymer is in the range of from 1700 to 15000 Da, preferably in the range of 10000 to 13000 Da. The present invention preferably requires polymers, which contains both EO and PO (propoxylate) parts, with the above structure. The ethoxylate (EO) part of the polymer is to preferably be from 10 to 80% of the total polymer, more preferably from 10 to 60% of the total polymer. Commercially available polymers which are preferably used in the composition of the present invention include Pluronic L-61, Pluronic F88, Pluronic F77, Pluronic F108, or Pluronic 127, more preferably Pluronic 127. The polymer is preferably present in 0.1 to 2wt%, more preferably 0.3 to 1.8wt%,

The composition of the invention includes a phosphate compound which is hexametaphosphate. Sodium hexametaphosphate has the structure as give below:

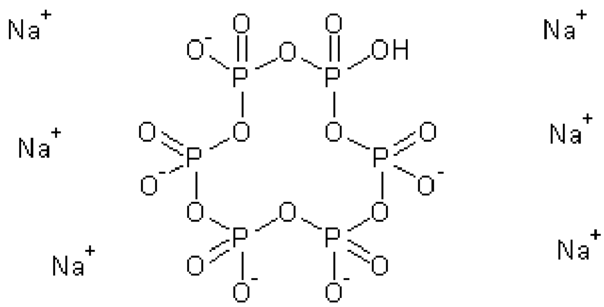

The hexametaphosphate is preferably included in 0.1 to 4%, more preferably 0.1 to 2.0% by weight of the total composition.

It is required as per the present invention that the weight ratio of polymer of formula 1 to hexametaphosphate is from 1:5 to 5:1, more preferably from 1:4 to 4:1. In a preferred aspect this ratio is from 1:3 to 3:1. It is particularly preferred if the weight ratio of polymer of formula 1 to hexametaphosphate is greater than 1:1.

It is preferred that the total amount of polymer and hexametaphosphate in the composition of the invention is from 0.1 to 5wt%, of the total composition.

Without wishing to be bound by theory, the present inventors believe that it is the synergistic interaction of the two ingredients of which the polymer modifies the hydroxyapatite surface of the tooth to provide a low interfacial energy to it, so as to repel stains from the surface and the calcium binding affinity of the phosphate to the tooth surface which at the specific weight ratios of the ingredients and the specifically chosen polymer structure within the chosen EO/PO ratio that provides the desired benefit of the present invention.

Compositions according to the invention are in the form of a product that is applied to the teeth as a spray.

The composition of the invention comprises an orally acceptable base. The orally acceptable base preferably comprises, a humectant, water, volatile alcohol or combinations thereof. The orally acceptable base preferably makes up 96 to 99.8% by weight of the composition Preferably the weight ratio of water to humectant is from 1:2 to 2:1, more preferably from 2:3 to 3:2.

A composition according to the invention (will generally contain further ingredients to enhance performance and/or consumer acceptability, in addition to the ingredients specified above.

Compositions according to the invention may comprise a polymeric deposition aid. Preferably the composition comprises acid anhydride polymers, particularly preferred are co-polymers of maleic anhydride with methyl vinylether, in which the anhydride moiety may be in a partially or fully hydrolysed or alcoholysed form. Preferred copolymers include Gantrez(R) polymers such as:

Gantrez S-95: molecular weight 216,000; free acid;
Gantrez S-96: molecular weight 700,000; free acid;
Gantrez S-97: molecular weight 1,500,000; free acid; and
Gantrez MS-955: molecular weight 1,060,000; calcium/sodium salt.

Particularly preferred co-polymers of maleic acid and methyl vinylether have a molecular weight of 1,000,000 or greater and an especially preferred material is Gantrez S-97.

Compositions according to the invention may comprise a tooth whitening agent. The whitening agent preferably comprises a green and/or a blue pigment. In the context of the present invention a pigment is generally understood to be a shade/material which is insoluble in the relevant medium, at the relevant temperature. This is in contrast to dyes which are soluble. In the context of this invention, the "relevant medium" is human saliva, the liquid medium in which the composition is used, at the temperature of the oral cavity during brushing of the teeth, i.e. up to 37 Degrees C. As a reasonable approximation, the relevant medium may be considered to be water and the relevant temperature to be 25 Degrees C.

Preferably the blue pigment is Pigment Blue 15, more preferably Pigment Blue 15:1, 15:2, 15:3, 15:4, 15:5 or 15:6, most preferably 15:1. A preferred pigment is blue pigment is Phthalocyanine Blue Pigment, CI No. 74160, blue covarine.

The preferred Green pigment is Phthalocyanine Green, preferably Phthalocyanine Green CI-74260.

Preferably the total level of pigment, in particular blue covarine in the total composition is from 0.0005 wt% to 0.5 wt%, more preferably from 0.001 to 0.1 wt% most preferably from 0.007 to 0.014 wt%.

Compositions according to the invention may comprise water-soluble or sparingly water-soluble sources of metal salts. Preferred are zinc ions such as zinc chloride, zinc acetate, zinc gluconate, zinc sulphate, zinc fluoride, zinc citrate, zinc lactate, zinc oxide, zinc monoglycerolate, zinc tartrate, zinc pyrophosphate and zinc maleate; also preferred are stannous ions such as stannous fluoride and stannous chloride.

Compositions according to the invention may comprise oral care enzyme systems such as hydrogen peroxide producing enzyme systems (e.g. the oxidoreductase enzyme glucose oxidase), amyloglucosidase, dextranase and/or mutanase, (optionally in the presence of zinc ion providing compounds and/or 8- hydroxyquinoline derivatives), lactoperoxidase, lactoferrin, lysozyme and mixtures thereof;

Compositions of the invention may comprise fluoride sources such as sodium fluoride, stannous fluoride, sodium monofluorophosphate, zinc ammonium fluoride, tin ammonium fluoride, calcium fluoride, cobalt ammonium fluoride and mixtures thereof;

Preferably the composition has a pH 6 to 9 at 20°C.

The composition according the invention will comprise further ingredients which are common in the art, such as:

antimicrobial agents, e.g. chlorhexidine, sanguinarine extract, metronidazole,
quaternary ammonium compounds, such as cetylpyridinium chloride; bis-guanides,
such as chlorhexidine digluconate, hexetidine, octenidine, alexidine; and halogenated bisphenolic compounds, such as 2,2' methylenebis-(4-chloro-6-bromophenol);
anti-inflammatory agents such as ibuprofen, flurbiprofen, aspirin, indomethacin etc.;
anti-caries agents such as sodium- and stannous fluoride, aminefluorides, sodium monofluorophosphate, sodium trimeta phosphate and casein;

plaque buffers such as urea, calcium lactate, calcium glycerophosphate and strontium polyacrylates;
vitamins such as Vitamins A, C and E;
plant extracts;
plant-derivable antioxidants such as flavonoid, catechin, polyphenol, and tannin compounds and mixtures thereof;
desensitising agents, e.g. potassium citrate, potassium chloride, potassium tartrate, potassium bicarbonate, potassium oxalate, potassium nitrate and strontium salts;
anti-calculus agents, e.g. alkali-metal pyrophosphates, hypophosphite-containing polymers, organic phosphonates and phosphocitrates etc.;
biomolecules, e.g. bacteriocins, antibodies, enzymes, etc.;
flavours, e.g. peppermint and spearmint oils;
proteinaceous materials such as collagen;
preservatives;
opacifying agents;
hyaluronic acid;
amino acids such as arginine;
colouring agents;
pH-adjusting agents;
sweetening agents;
pharmaceutically acceptable carriers, e.g. starch, sucrose, water or water/alcohol systems etc.;
surfactants, such as anionic, nonionic, cationic and zwitterionic or amphoteric surfactants;
Humectants such as glycerol, sorbitol, propyleneglycol, xylitol, lactitol etc.;
binders and thickeners such as sodium carboxymethyl-cellulose, hydroxyethyl cellulose (Natrosol®), xanthan gum, gum arabic etc. as well as synthetic polymers such as polyacrylates and carboxyvinyl polymers such as Carbopol®;
polymeric compounds which can enhance the delivery of active ingredients such as antimicrobial agents can also be included;
buffers and salts to buffer the pH and ionic strength of the oral care composition; and other optional ingredients that may be included are e.g. bleaching agents such as peroxy compounds e.g. potassium peroxydiphosphate, effervescing systems such as sodium bicarbonate/citric acid systems, colour change systems, and so on.

The invention will now be illustrated by the following non-limiting examples.

Examples of the invention are represented by a number, comparative Examples are illustrated by a letter.

## Examples

### Example 1 and Example A

Two sprays were produced as in Table 1

**Table 1**

| Ingredient | Example 1 | Example A |
|---|---|---|
| Sorbitol | 50.00 | 52.00 |
| Sodium Saccharin | 0.27 | 0.27 |
| Sodium hexametaphosphate | 0.50 | 0 |
| Pluronic F 127 | 1.50 | 0 |
| Cremaphor | 2.50 | 2.50 |
| Flavour | 0.50 | 0.50 |
| Water | 44.73 | 44.73 |
| Total | 100 | 100 |

### Testing Method

Extracted teeth were cleaned with a silica-based toothpaste and placed in sterile human pooled saliva for 2 hours to allow a pellicle to form. Baseline tooth colour was measured using a colorimeter. The teeth were gently dabbed

with a tissue to remove excess saliva and each tooth was then given 2 spray applications of either the anti-stain formulation (containing pluronic F127 and sodium hexametaphosphate) or the placebo formulation. The teeth were left for 30 seconds before placing in a stain solution at 25°c (1:1:1 black coffee solution, black tea solution, red wine) with gentle agitation (75rpm). The teeth were removed at each time point, rinsed in 50ml of water, the colour remeasured and then placed back in the stain. Total colour change (ΔE) from baseline and hence level of stain formation was calculated following different timepoints using:

$$\Delta E = \sqrt{[(L^*_0 - L^*_t)^2 + (a^*_0 - a^*_t)^2 + (b^*_0 - b^*_t)^2]}$$

Where $L^*_0$ and $L^*_t$ are L* values at baseline and time t respectively; $a^*_0$ and $a^*_t$ are a* values at baseline and time t respectively, and $b^*_0$ and $b^*_t$ are b* values at baseline and time t.

**Results**

Mean ΔE (s.e.) values for each treatment group (n=15) after various time points in the staining solution are shown below in Table 2. The product differences were of statistical significance (Student's t-test, $p<0.05$) at each time point.

**Table 2**

| Formulation | Mean ΔE (s.e.) values | | |
|---|---|---|---|
| | 1.5hr | 3hr | 4.5hr |
| Example 1 | 1.12 ±0.12 | 1.38 ±0.56 | 1.31 ±0.15 |
| Example A | 1.97 ±0.10 | 2.85 ±0.90 | 3.25 ±0.20 |

The data in the table 2 indicates that oral care compositions as per the invention (Example 1) provides vastly superior anti-staining efficacy (as evident by the low ΔE values) as compared to the comparative Example (Example A).

**Examples 2,3 and 4**

**Table 3**

| Ingredient | Example 2 | Example 3 | Example 4 | Example A |
|---|---|---|---|---|
| Sorbitol | 50.00 | 50.00 | 50.00 | 52.00 |
| Sodium Saccharin | 0.27 | 0.27 | 0.27 | 0.27 |
| Sodium hexametaphosphate | 0.50 | 0.50 | 0.50 | 0 |
| Pluronic F 127 | 1.50 | 1.50 | 1.50 | 0 |
| Blue covarine | 0.005 | 0.01 | 0.015 | 0 |
| Cremaphor | 2.50 | 2.50 | 2.50 | 2.50 |
| Flavour | 0.50 | 0.50 | 0.50 | 0.50 |
| Water | 44.73 | 44.73 | 44.73 | 44.73 |
| Total | 100 | 100 | 100 | 100 |

The method of evaluation for Examples A and 2-4 os as for Example 1, but with exposure time of 10s following spray before an additional colour measurement was taken (instant whitening effect) and then immersion in the stain solution for 1.5 and 3 hours before colour measurements (anti-stain effect). The instant whitening effect is described in terms of changes in the tooth Whiteness Index (WIO) from baseline and the subsequent stain formation in terms of ΔE from baseline. The results are shown in Table 4.

**Table 4**

| Formulation | Instant Whitening Effect Mean ΔWIO (s.e) | Stain Formation Mean ΔE (s.e) | |
|---|---|---|---|
| | 0.0hr | 1.5hr | 3.0hr |
| Example A | 0.1 (0.4) A | 6.0 (0.3) A | 7.9 (0.3) A |
| Example 2 | 4.8 (0.7) B | 2.7 (0.5) B | 2.9 (0.5) B |
| Example 3 | 6.7 (0.9) B | 2.4 (0.4) B | 1.6 (0.3) C |
| Example 4 | 12.3 (0.8) C | 2.9 (0.3) B | 3.5 (0.3) B |

Values with different letters within each time point indicate statistically significant differences between treatment groups ($p<0.05$, ANOVA, Tukey-Kramer).

The data in Table 3 indicates that the addition of blue covarine at different levels to the spray formulation (Example 1) gives instant whitening benefits superior to the comparative formulation (Example A) as evident by the greater and significant change in WIO values. In addition, the blue covarine containing spray formulation provides vastly superior anti-staining efficacy (as evident by the significantly lower ΔE values) as compared to the comparative Example (Example A).

**Claims**

1. An oral care spray composition comprising:

   (a) 0.1 to 2 wt% of the total composition of polymer having the structure

   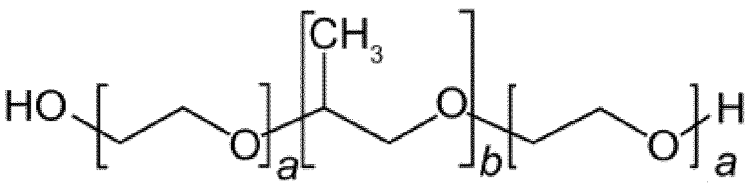

   in which a is independently selected from 2 to 130 and *b* is 15 to 67 and the molecular weight of the polymer is from 1,700 to 15,000 Da.
   (b) hexametaphosphate; and
   (c) an orally acceptable base;

   wherein the weight ratio of the polymer to hexametaphosphate is from 1:5 to 5:1.

2. A composition as claimed in claim 1 wherein the weight ratio of polymer to hexametaphosphate is from 1:4 to 4:1.

3. A composition as claimed in any one of the preceding claims wherein the molecular weight of the polymer is from 10,000 to 13,000 Da.

4. A composition as claimed in any one of the preceding claims comprising from 0.1 to 4wt% of the total composition of hexametaphosphate.

5. A composition according to any preceding claim in which the total amount of polymer and hexametaphosphate is from 0.1 to 5wt% of the total composition.

6. A composition according to any preceding claim in which the composition further comprises a blue pigment, preferably blue covarine.

7. A composition according to claim 7 in which the level of blue pigment is from 0.001 to 0.1 wt% of the total composition,

preferably from 0.007 to 0.014 wt%.

8. A composition as claimed in any one of the preceding claims wherein the orally acceptable base comprises a humectant, water, volatile alcohol or combinations thereof.

9. A composition as claimed in any one of the preceding claims comprising from 96 to 99.8% of orally acceptable base.

10. A composition as claimed in claim 8 or claim 9 wherein the humectant is a polyhydric alcohol preferably sorbitol or glycerol.

11. A composition according to any one of claims 8 to 10 in which the weight ratio of water to humectant is from 1:2 to 2:1.

12. A composition according to any preceding claim in which has a pH at 20°C from 6 to 9.

13. A method of minimising or preventing the staining of teeth comprising the steps of applying the composition as claimed in any one of the preceding claims on to a tooth surface.

14. Cosmetic use of a composition according to any one of claims 1 to 11 to mitigate the staining of teeth.

**Patentansprüche**

1. Mundpflege-Sprayzusammensetzung, umfassend:

(a) 0,1 bis 2 Gew.-% der Gesamtzusammensetzung an Polymer der Struktur

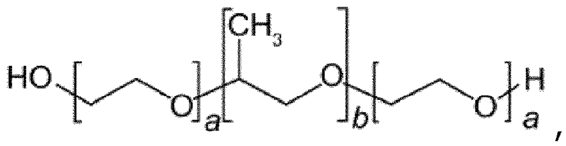

in der a unabhängig unter 2 bis 130 ausgewählt ist und b 15 bis 67 beträgt und das Molekulargewicht des Polymers 1.700 bis 15.000 Da beträgt;
(b) Hexametaphosphat; und
(c) eine oral akzeptable Basis;

wobei das Gewichtsverhältnis von Polymer zu Hexametaphosphat 1:5 bis 5:1 beträgt.

2. Zusammensetzung, wie im Anspruch 1 beansprucht, wobei das Gewichtsverhältnis von Polymer zu Hexametaphosphat 1:4 bis 4:1 beträgt.

3. Zusammensetzung, wie in irgendeinem der vorhergehenden Ansprüche beansprucht, wobei das Molekulargewicht des Polymers 10.000 bis 13.000 Da beträgt.

4. Zusammensetzung, wie in irgendeinem der vorhergehenden Ansprüche beansprucht, umfassend 0,1 bis 4 Gew.-% der Gesamtzusammensetzung an Hexametaphosphat.

5. Zusammensetzung nach irgendeinem vorhergehenden Anspruch, in der die Gesamtmenge an Polymer und Hexametaphosphat 0,1 bis 5 Gew.-% der Gesamtzusammensetzung beträgt.

6. Zusammensetzung nach irgendeinem vorhergehenden Anspruch, wobei die Zusammensetzung ferner ein blaues Pigment, vorzugsweise Blue Covarine, umfasst.

7. Zusammensetzung nach Anspruch 6, in der der Gehalt an blauem Pigment 0,001 bis 0,1 Gew.-% der Gesamtzusammensetzung, vorzugsweise 0,007 bis 0,014 Gew.-%, beträgt.

8. Zusammensetzung, wie in irgendeinem der vorhergehenden Ansprüche beansprucht, wobei die oral akzeptable

Basis ein Feuchthaltemittel, Wasser, flüchtigen Alkohol oder Kombinationen davon umfasst.

**9.** Zusammensetzung, wie in irgendeinem der vorhergehenden Ansprüche beansprucht, umfassend 96 bis 99,8% der oral akzeptablen Basis.

**10.** Zusammensetzung, wie im Anspruch 8 oder Anspruch 9 beansprucht, wobei das Feuchthaltemittel ein mehrwertiger Alkohol, vorzugsweise Sorbitol oder Glycerol, ist.

**11.** Zusammensetzung nach irgendeinem der Ansprüche 8 bis 10, wobei das Gewichtsverhältnis von Wasser zu Feuchthaltemittel 1:2 bis 2:1 beträgt.

**12.** Zusammensetzung nach irgendeinem vorhergehenden Anspruch, wobei der pH-Wert bei 20°C 6 bis 9 beträgt.

**13.** Verfahren zum Minimieren oder Verhindern des Verfärbens von Zähnen, umfassend die Schritte des Auftragens der Zusammensetzung, wie in irgendeinem der vorhergehenden Ansprüche beansprucht, auf eine Zahnoberfläche.

**14.** Kosmetische Verwendung einer Zusammensetzung nach irgendeinem der Ansprüche 1 bis 11 zur Milderung des Verfärbens von Zähnen.

**Revendications**

**1.** Composition de pulvérisation pour le soin oral comprenant :

(a) de 0,1 à 2 % en masse de la composition totale de polymère ayant la structure

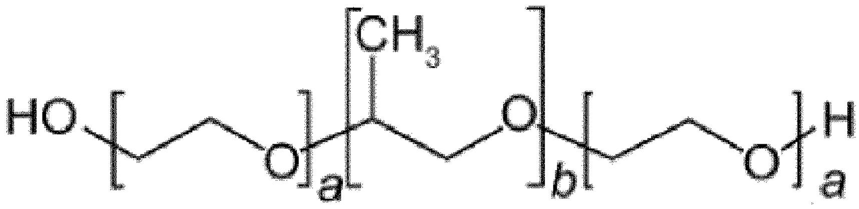

dans laquelle *a* est indépendamment choisi de 2 à 130 et *b* est de 15 à 67 et la masse moléculaire du polymère est de 1 700 à 15 000 Da.
(b) de l'hexamétaphosphate ; et
(c) une base oralement acceptable ;

dans laquelle le rapport en masse du polymère à hexamétaphosphate est de 1:5 à 5:1.

**2.** Composition selon la revendication 1, dans laquelle le rapport en masse de polymère à hexamétaphosphate est de 1:4 à 4:1.

**3.** Composition selon l'une quelconque des revendications précédentes, dans laquelle la masse moléculaire du polymère est de 10 000 à 13 000 Da.

**4.** Composition selon l'une quelconque des revendications précédentes comprenant de 0,1 à 4 % en masse de la composition totale d'hexamétaphosphate.

**5.** Composition selon l'une quelconque des revendications précédentes, dans laquelle la quantité totale de polymère et hexamétaphosphate est de 0,1 à 5 % en masse de la composition totale.

**6.** Composition selon l'une quelconque des revendications précédentes, dans laquelle la composition comprend de plus un pigment bleu, de préférence de la covarine bleue.

**7.** Composition selon la revendication 7, dans laquelle la teneur en pigment bleu est de 0,001 à 0,1 % en masse de la composition totale, de préférence de 0,007 à 0,014 % en masse.

**8.** Composition selon l'une quelconque des revendications précédentes, dans laquelle la base oralement acceptable comprend un humectant, de l'eau, un alcool volatil ou des combinaisons de ceux-ci.

**9.** Composition selon l'une quelconque des revendications précédentes comprenant de 96 à 99,8 % de base oralement acceptable.

**10.** Composition selon la revendication 8 ou revendication 9, dans laquelle l'humectant est un alcool polyvalent de préférence du sorbitol ou glycérol.

**11.** Composition selon l'une quelconque des revendications 8 à 10, dans laquelle le rapport en masse d'eau à humectant est de 1:2 à 2:1.

**12.** Composition selon l'une quelconque des revendications précédentes qui présente un pH à 20°C de 6 à 9.

**13.** Procédé de minimisation ou de prévention de coloration des dents comprenant les étapes d'application de la composition selon l'une quelconque des revendications précédentes sur une surface de dent.

**14.** Utilisation cosmétique d'une composition selon l'une quelconque des revendications 1 à 11 pour mitiger la coloration de dents.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2014377194 A
- US 2003124065 A
- US 5057309 B